# EUROPEAN PATENT SPECIFICATION

(11) **EP 4 441 484 B1**
(45) Date of publication and mention of the grant of the patent: **22.07.2026**
(21) Application number: 23717650.8
(22) Date of filing: 24.03.2023
(51) Int. Cl.: G01N 29/024, G01N 29/032, G01N 29/22, G01N 29/28, G01N 29/32, A01J 11/16, G01N 33/02, G01N 33/15

(54) **AN ULTRASONIC MEASURING APPARATUS FOR INLINE MEASURING THE HOMOGENIZING DEGREE OF A FLUID PRODUCT IN A DISPERSING UNIT**
ULTRASCHALL-MESSGERÄT ZUR INLINE-MESSUNG DES HOMOGENISIERUNGSGRADES EINES FLÜSSIGEN PRODUKTS IN EINER DISPERGIEREINHEIT
APPAREIL DE MESURE À ULTRASONS POUR MESURE EN LIGNE DU DEGRÉ D'HOMOGÉNÉISATION D'UN PRODUIT FLUIDE DANS UNE UNITÉ DE DISPERSION

(30) Priority: 23.02.2023 IT 202300003195
(43) Date of publication of application: 09.10.2024
(73) Proprietor: GEA Mechanical Equipment Italia S.p.A., 43123 Parma (IT)
(72) Inventor: MAGGI, Leonardo, 43124 Parma (IT); TONELLI, Annachiara, 43044 Collecchio (Parma) (IT); BENASSI, Massimiliano, 43045 Fornovo di Taro (Parma) (IT); BICZ, Agnieszka, 52-428 Wroclaw (PL); BICZ, Wieslaw, 52-428 Wroclaw (PL)
(74) Representative: Dondi, Silvia
(86) International application number: PCT/IB2023/052951
(87) International publication number: WO 2024/175967

(56) References cited:
- CN-A- 103 323 064
- CN-A- 108 414 039
- CN-A- 111 189 500
- DE-A1- 3 319 922
- US-A1- 2004 112 121
- US-A1- 2009 272 190
- ANONYMOUS: "Piezoelectric transducer design | Ultrasonic Resonators", 6 January 2022 (2022-01-06), pages 1 - 55, XP093084546, Retrieved from the Internet <URL:http://www.ultrasonic-resonators.org/design/transducers/transducer_design.html> [retrieved on 20230921]

## Description

### Technical field

The present invention relates to a measuring apparatus for inline measuring the homogenizing degree of a fluid product in a dispersing unit. The invention proposed here is used in the food industry, in particular in the dairy sector, or in the chemical, pharmaceutical or cosmetic industry. The invention can also be used in manufacturing areas where homogenization is a step of the production process.

Consider, for example, the production of carbon-based nanostructured materials, such as graphene and carbon nanotubes or cellular breakdown of yeasts, algae, or microorganisms for the production of intracellular material.

### Background art

Even though in varying, currently known embodiments, a homogenizing apparatus comprises a high-pressure pump and a homogenizing valve which acts on the fluid products in order to:
- crush the particles of the fluid to make their dimensions uniform, reducing the average size and the variance of the distribution in order to stabilize the product and to increase its shelf-life in the case of emulsions;
- break the cell membranes in order to facilitate the extraction of the active ingredients in the case of pharmaceutical applications;
- modify the structure of the particles in the case of chemical applications and cellulose or unicellular organisms.

For most products, the main parameters defining the level of homogenization are the average particle size and standard deviation.

In the known solutions, the pressure of the homogenizing apparatus is adjusted as described here below.

First, the optimum average particle size and standard deviation for a given fluid product are experimentally determined. Then, it is applied a pressure value necessary to obtain particles showing the calculated parameters. Maintaining the pressure is achieved by one of the following methods:
- periodically adjusting the pressure value by manual interventions;
- using a feedback system which receives as input the pressure level detected in the fluid product and forces the output to follow a set point.

Both methods act directly on the homogenizing pressure.

Nevertheless, the homogenizing pressure cannot be considered a reliable real-time indicator of the actual homogenizing level. In fact, the pressure and the fluid product being equal, different homogenizing levels can be obtained, for example due to modifications occurring in the new product fed to the apparatus and/or due to wear of the components.

The known methods cannot track differences in the fluid product fed to the homogenizing apparatus.

In addition, the known methods do not consider changes caused in the fluid product by non-ideal components.

These situations are usually dealt with by designing a homogenizing apparatus able to assure a homogenizing level above a pre-established threshold, which corresponds to the most adverse conditions.

Having an oversized homogenizing apparatus raises risks of mechanical stress for some components, which may decrease their lifetime.

In addition, applying pressure values which are higher than requested is energy consuming.

Finally, applying pressure values which are higher than requested does not assure to reach the desired homogenizing level, as it causes an increase in temperature that may damage the fluid product. Part of the product could even be discarded due to thermal damage.

Document EP 1121973 A1 discloses a system for the supervision of a dispersion produced in a dispersing unit, in particular the homogenizing degree thereof, wherein it is proposed to couple at least one measurement sensor to the dispersing unit to achieve an in-line measurement of the homogenizing degree for the dispersion.

The sensor used in this document may be an ultrasonic sensor immersed in the fluid product or in contact with it. Thus, the sensor easily gets dirty and unreliable. In addition, it is more prone to be damaged by high temperature of the fluid product.

Document US 2009/0272190 A1 discloses a non-invasive method for measuring parameters in a fluid flowing thorugh a conduit, where the method comprises trasmitting ultrasound signals through the fluid.

Document US 2004/0112121 A1 discloses a method and a device for monitoring and controlling critical parameters, such as particles, biomolecules, and viscosity, in industrial pharmaceutical, food and chemical processes, again based on ultrasound signals transmission.

Document DE 33 19 922 A1 discloses a method for controlling processes in which a disperse phase is involved.

Specific designs of ultrasonic transducers are disclosed in documents CN 103 323 064 A and CN 111 189 500 A.

Another type of transducer is disclosed in the online publication titled *"Piezoelectric transducer design",* which may be retrieved from the internet at *http:*//*www.ultrasonic-resonators.org*/*design*/*transducers*/*transducer_design.html.*

Document CN 108 414 039 A discloses a water temperature detecting method and a water flow sensor of a water heater.

### Disclosure of the invention

In this context, the object of the present invention is to propose a measuring apparatus for inline measuring the homogenizing degree of a fluid product in a dispersing unit, which overcomes the problems of the prior art cited above.

In particular, the object of the present invention is to propose a measuring apparatus for inline measuring the homogenizing degree of a fluid product in a dispersing unit, which achieves a higher measurement reliability than the known solutions.

A further object of the present invention is to provide a measuring apparatus for inline measuring the homogenizing degree of a fluid product in a dispersing unit, which does not affect the fluid product.

Another object of the present invention is to propose a measuring apparatus for inline measuring the homogenizing degree of a fluid product in a dispersing unit, which is robust, less likely to wear over time and easy to clean.

Another object of the present invention is to propose a measuring apparatus for inline measuring the homogenizing degree of a fluid product in a dispersing unit, in which precautionary maintenance operations can be scheduled in a more efficient way.

The stated technical task and specified aims are achieved by a measuring apparatus according to claim 1.

In one embodiment of the invention, the ultrasound sensors are glued to the first tube.

Preferably, the first tube is made of a plastic material and the second tube is made of a metal material.

More preferably, the first tube is made of PEEK or PTFE.

The second tube is made of stainless steel.

### Brief description of drawings

Further characteristics and advantages of the present invention will more fully emerge from the non-limiting description of a preferred but not exclusive embodiment of a measuring apparatus for inline measuring the homogenizing degree of a fluid product in a dispersing unit, as illustrated in the accompanying drawings in which:
- figures 1 and 2 illustrate two different embodiments of a measuring apparatus for inline measuring the homogenizing degree of a fluid product, according to the present invention;
- figure 3 shows the waveforms of measured signals received by four sensors in an experimental test carried out for choosing the best material for the first tube of the measuring apparatus;
- figure 4 illustrates a block diagram of the measuring apparatus of figures 1-2 at an output of a dispersing unit.

### Detailed description of preferred embodiments of the invention

With reference to the figures, the number 100 denotes a dispersion unit, The dispersion unit 100 has an input 100a and an output 101b for a fluid product.

For example, the dispersion unit 100 is a high-pressure homogenizer comprising a volumetric piston pump and a homogenizing valve arranged downstream of the volumetric piston pump.

In particular, the high-pressure homogenizer 100 operates with pressure up to 4000 bar.

Number 1 denotes a measuring apparatus for inline measuring the homogenizing degree of a fluid product in the dispersion unit 100.

The measuring apparatus 1 comprises a tubular device 2 arranged at the output 100b of the dispersion unit 100.

The tubular device 2 has an inlet 2a receiving the homogenized fluid product from the dispersion unit 100, and an outlet 2b.

The tubular device 2 has an internal cavity 3 for the passage of the fluid product received from the dispersion unit 100.

The internal cavity 3 extends from the inlet 2a to the outlet 2b of the tubular device 2.

The diameter of the internal cavity 3 at the inlet 2a and at the outlet 2b of the tubular device 2 is substantially the same.

Originally, the internal cavity 3 has a variable diameter across the length of the tubular device 2.

In particular, the internal cavity 3 has at least one first tract 31 with a tapering development from the inlet 2a towards the outlet 2b of the tubular device 2.

In other words, the first tract 31 has a convergent shape from the inlet 2a towards the outlet 2b of the tubular device 2.

Preferably, the first tract 31 of the internal cavity 3 is formed by a plurality of substantially cylindrical portions 310 which are joined by frusto-conical portions 311.

The cylindrical portions 310 have corresponding diameters that decrease from the inlet 2a towards the outlet 2b of the tubular device 2.

In the illustrated embodiment, the first tract 31 comprises five cylindrical portions 310 which are joined by four frusto-conical portions 311.

The internal cavity 3 preferably has a second tract 32 originating from the first tract 31 and ending at the outlet 2b of the tubular device 2.

The second tract 32 has a tapering development from the outlet 2b of the tubular device 2 towards the first tract 31.

In other words, the second tract 32 has a convergent shape from the outlet 2b of the tubular device 2 towards the first tract 31.

In particular, the second tract 32 consists of a frusto-conical portion 321 connecting the first tract 31 to the outlet 2b of the tubular device 2.

In particular, the last cylindrical portion 310 of the first tract 31 is connected to the frusto-conical portion 321 of the second tract 32.

The measuring apparatus 1 further comprises a plurality of ultrasound sensors 4 mounted on the tubular device 2 and configured to detect ultrasound waves.

According to the illustrated embodiment, the tubular device 2 comprises a first tube 5 and a second tube 6.

The first tube 5 is coaxial with and internally arranged inside the second tube 6.

The first tube 5 delimits the internal cavity 3. In particular, the internal cavity 3 is delimited by an internal surface 5a of the first tube 5.

In particular, the internal surface 5a of the first tube 5 partially has a tapering development from the inlet 2a towards the outlet 2b of the tubular device 2 so as to delimit the corresponding tapering development of the first tract 31 of the internal cavity 3.

According to the invention, the ultrasound sensors 4 are arranged between the first tube 5 and the second tube 6.

Preferably, the ultrasound sensors 4 are distributed along the length of the tubular device 2.

According to the embodiments illustrated herewith, the first tube 5 has a plurality of housings 7 for receiving the ultrasound sensors 4.

Preferably, the housings 7 are obtained as inward recesses of an external surface 5b of the first tube 5.

According to one embodiment of the invention, the ultrasound sensors 4 are glued to the first tube 5. In particular, they are placed inside the housings 7 and glued to the external surface 5b of the first tube 5.

For example, the ultrasound sensors 4 are screwed externally to the second tube 6 so as to arrive close to the first tube 5.

Preferably, in this embodiment an ultrasound gel is deposited between the ultrasound sensors 4 and the first tube 5 for assuring the transmission of the signals.

Preferably, the measuring apparatus 1 has an electrical connector mounted on the second tube 6. The electrical connector serves for the connections of the ultrasound sensors 4.

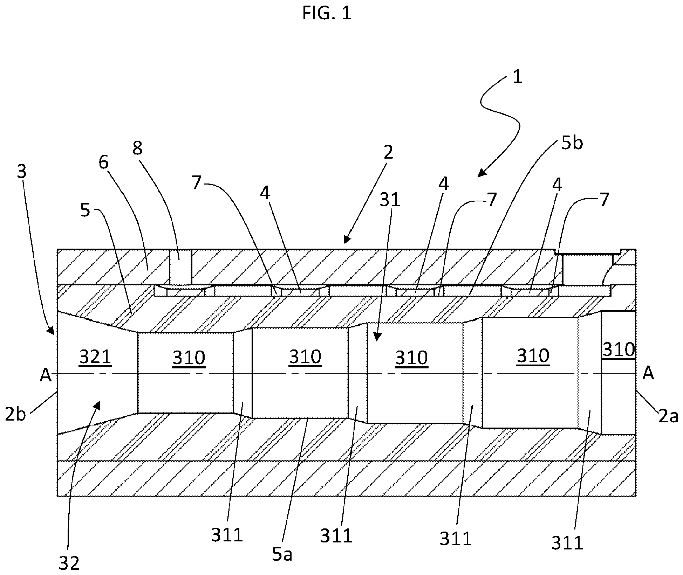

According to an embodiment of the invention, illustrated in figure 1, each cylindrical portion 310 is coupled to an ultrasound sensor 4. Said ultrasound sensor 4 is arranged inside a housing 7 obtained in a section of the first tube 5 surrounding the corresponding cylindrical portion 310.

In this embodiment, referred to as "Pulse Echo", the ultrasound sensors 4 are configured to generate and detect ultrasound waves. In other words, the ultrasound sensors 4 act as emitters and receivers.

The waves generated by the ultrasound sensors 4 pass through the fluid product flowing in the internal cavity 3 and are reflected back by the second tube 6, which is made of metal.

Alternatively, reflectors may be mounted on a side opposite to the housings 7 with respect to the internal cavity 3 so as to increase the reflection of the ultrasound waves.

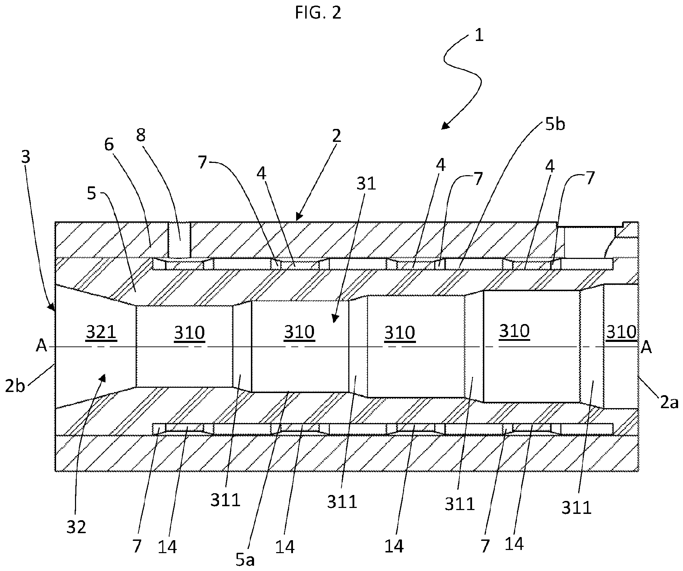

According to another embodiment of the invention, illustrated in figure 2, each cylindrical portion 310 is coupled to one of the ultrasound sensors 4 previously mentioned (which will be referred here as "receiver"), and to another ultrasound sensor 14 that is configured to generate ultrasound waves (and will be referred as "emitter"). The receivers 4 and the emitters 14 are respectively arranged in two housings 7 oppositely located with respect to the internal cavity 3. The two housings 7 are obtained in a section of the first tube 5 surrounding the corresponding cylindrical portion 310, but they are obtained on different sides with respect to the flow of the fluid product.

In this embodiment, referred to as "Through-Transmission", the waves generated by the emitters 14 pass through the fluid product flowing in the internal cavity 3 and are detected by the receivers 4.

According to an aspect of the invention, the first tube 5 is made of a plastic material. Thus, ultrasound waves can pass through the first tube 5.

In a preferred embodiment, the first tube 5 is made of PEEK. Alternatively, the first tube 5 is made of PTFE.

An experimental test has been carried out for choosing the best material for the first tube 5.

The test consisted in creating four different tubular sample portions, each formed by a different material for simulating the first tube 5.

Each sample portion has its own ultrasound sensor, which is a piezoelectric ceramic sensor with resonance frequency of 5 MHz. The ultrasound sensor is screwed to the corresponding sample portion.

These are the four tubular sample portions created for the test:
- 1^{st} sample portion: tube made of AISI 316L, with a thickness of 2 mm (from the internal cavity to the corresponding screwed sensor);
- 2^{nd} sample portion: tube made of PTFE, with a thickness of 10 mm (from the internal cavity to the corresponding screwed sensor);
- 3^{rd} sample portion: tube made of PEEK, with a thickness of 10 mm (from the internal cavity to the corresponding screwed sensor);
- 4^{th} sample portion: tube made of AISI 316L, with a thickness of 4 mm (from the internal cavity to the corresponding screwed sensor).

In practice, the sample portions are simplified versions of the tubular device 2 (with only the first tube 5), with a Pulse echo arrangement, where the ultrasound sensors act both as emitters and receivers.

The thicknesses of the first three samples are different. They have been chosen to obtain an adequate value of mechanical resistance to withstand an internal pressure of 40 bar inside the tube. The fluid inside the cavity is tap water at ambient temperature.

The 3^{rd} sample (PEEK) resulted the best choice since the signal after the first reflection is well distinguished over the noise.

PEEK is the best choice since it combines a high mechanical resistance with chemical inertia, suitability with food grade / pharmaceutical substances, resistance to high sterilization temperatures, transparency to ultrasonic waves.

In addition, the 4^{th} sample has been inserted to compare how the signal is transmitted in AISI 316L having a higher thickness (i.e., double) than the 1^{st} sample. In the 4^{th} sample the higher thickness of AISI 316L significantly attenuates the ultrasonic signal transmission, and the first reflection of the signal is affected by noise.

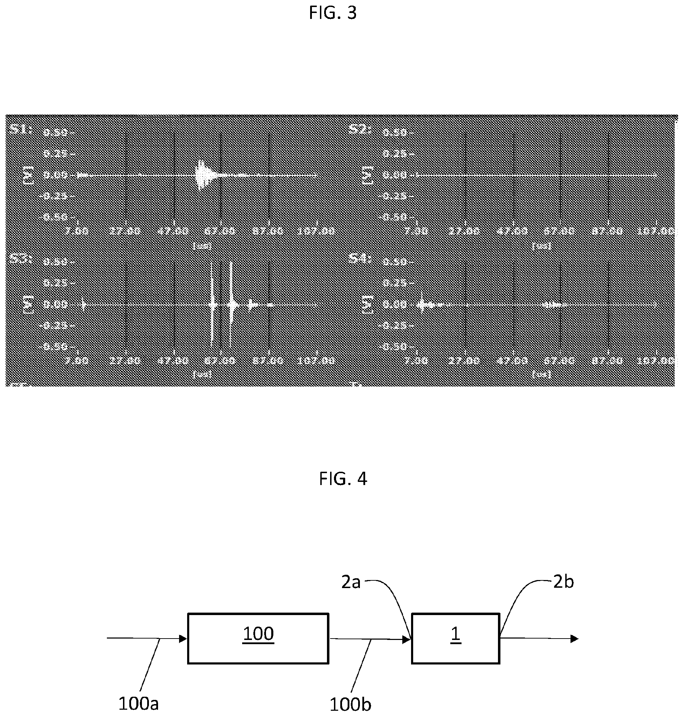

The measured signals received by the four sensors are reported in figure 3 for a quick comparison, where:
- S1 refers to the 1^{st} sample portion;
- S2 refers to the 2^{nd} sample portion;
- S3 refers to the 3^{rd} sample portion;
- S4 refers to the 4^{th} sample portion.

The second tube 6 is made of a metal material. For example, the second tube 6 is made of stainless steel. Thus, the ultrasound waves are reflected by the second tube 6 and maintained within the tubular device 2.

According to the invention, the tubular device 2 comprises a heat exchanger 8 for cooling down the ultrasonic sensors 4.

Preferably, the heat exchanger 8 is a conduit for the passage of a cooling fluid.

Preferably, the conduit 8 passes through the tubular device 2 for its whole length and has a main tract that is parallel to a longitudinal axis A-A of the tubular device 2.

According to one embodiment of the invention, the conduit 8 has an inlet obtained in the second tube 6 in correspondence of the housing 7 which is located closer to the inlet 2a of the tubular device 2, and an outlet obtained in the second tube 6 in correspondence of the housing 7 which is located closer to the outlet 2b of the tubular device 2.

Alternatively, the inlet of the conduit 8 is closer to the outlet 2b of the tubular device 2, and the outlet of the conduit 8 is closer to the inlet 2a of the tubular device 2. In particular, the conduit 8 is fed with air having a temperature of 5°- 40°C.

The air flowing within the conduit 8 serves for maintaining under control the temperature of the ultrasound sensors 4, independently from the temperature of the fluid product flowing in the internal cavity 3.

This is advantageous both during the normal working of the dispersing unit 100 and during sterilization cycles, where the tubular device 2 can reach high temperature (for example 130°C) which may affect the reliability of the ultrasound sensors 4 or even damage them.

In addition, in the embodiment in which the ultrasound sensors 4 are glued to the first tube 5, a high temperature could unglue the sensors 4 from the first tube 5, and an air layer could originate between the sensors 4 and the first tube 5, thus interrupting signal transmissions.

The ultrasound sensors 4 are configured to receive ultrasound waves having a frequency comprised between 2 MHz and 20 MHz.

Preferably, the ultrasound sensors 4 are chosen to work at different frequencies so as to detect the ultrasound waves passing in fluid products with different acoustic impedances.

The signal detected by the ultrasound sensors 4 may be used to control the pressure of the fluid product in the dispersion unit 100.

The characteristics of the measuring apparatus for inline measuring the homogenizing degree of a fluid product in a dispersing unit, according to the present invention emerge clearly from the above description, as do the advantages.

Since the ultrasound waves are distributed along the whole length of the tubular device, detection is simultaneously carried out in multiple measurement points of the flow of fluid product.

Thus, this detection is more reliable than detecting in a single point or a single specimen of fluid product. Redundancy is also advantageous in case of damage of one or more sensors.

In addition, arranging the ultrasound sensors on the external surface of the first tube avoids a direct contact with the fluid product which flows in the internal cavity. This results in a beneficial hygienic design of the tubular device.

In addition, in the embodiment in which the ultrasound sensors are glued to the first tube, there is substantially no air in the housings for the ultrasound sensors. This assures a good quality of signals, since air would attenuate ultrasound waves.

Furthermore, in the embodiment in which the ultrasound sensors are glued to the first tube, the ultrasound sensors are substantially maintained in their positions during the assembling and disassembling operations of the tubular device. This is assured by their arrangement in the inward recesses of the external surface of the first tube. Indeed, this arrangement also protects the ultrasound sensors from damages.

In the embodiment in which the ultrasound sensors are glued to the first tube, the ultrasound sensors are not mechanically coupled to the second tube, which is made of stainless steel. Thus, there is no risk of accumulation of particles (i.e., dust) between the second tube and the ultrasound sensors. In addition, the second tube is almost smooth, with only one electrical connector. The external surface of the second tube is thus easy to clean.

## Claims

1. A measuring apparatus (1) for inline measuring the homogenizing degree of a fluid product in a dispersing unit (100), said measuring apparatus comprising:
- a tubular device (2) arrangeable at an output (100b) of the dispersing unit (100), said tubular device (2) having an internal cavity (3) for the passage of the fluid product received from the dispersing unit (100), said tubular device (2) comprising a first tube (5) and a second tube (6), the first tube (5) being coaxial with and internally arranged inside the second tube (6), said internal cavity (3) being delimited by an internal surface (5a) of the first tube (5), said tubular device (2) further comprising a heat exchanger which is a conduit (8) for the passage of a cooling fluid, said conduit (8) passing through the tubular device (2) for its whole length and having a main tract that is parallel to a longitudinal axis (A-A) of the tubular device (2);
a plurality of ultrasound sensors (4) distributed along the length of the tubular device (2) and
being arranged between the first tube (5) and the second tube (6), said first tube (5) having a plurality of housings (7) for receiving said ultrasound sensors (4), said housings (7) being obtained as inward recesses of an external surface (5b) of the first tube (5), said plurality of ultrasound sensors being cooled down by the heat exchanger,
wherein said internal cavity (3) has a variable diameter across the length of the tubular device (2), the internal cavity (3) having at least one first tract (31) having a convergent shape from an inlet (2a) of the tubular device (2) towards an outlet (2b) of the tubular device (2), said internal cavity (3) having also a second tract (32) originating from the first tract (31) and ending at the outlet (2b) of the tubular device (2), said second tract (32) having a tapering development from the outlet (2b) of the tubular device (2) towards said first tract (31), said second tract (32) consisting of a frusto-conical portion (320) connecting the first tract (31) to the outlet (2b) of the tubular device (2),
wherein the first tract (31) of the internal cavity (3) is formed by a plurality of substantially cylindrical portions (310) which are joined by frusto-conical portions (311), said cylindrical portions (310) having corresponding diameters that decrease from the inlet (2a) towards the outlet (2b) of the tubular device (2),
each cylindrical portion (310) being either:
- coupled to one of said ultrasound sensors (4) acting both as emitter and receiver of ultrasound waves and arranged inside one of said housings (7) obtained in a section of the first tube (5) surrounding said corresponding cylindrical portion (310), or
- coupled to one of said ultrasound sensors (4) acting as receiver of ultrasound waves and to another of said ultrasound sensors (14) acting as emitter of ultrasound waves, said emitter and said receiver being respectively arranged in two housings (7) of said housings (7) oppositely located with respect to the internal cavity (3) and being obtained in a section of the first tube (5) surrounding the corresponding cylindrical portion (310), but on different sides with respect to the flow of the fluid product.

2. The measuring apparatus (1) according to claim 1, wherein the ultrasound sensors (4) are glued to the first tube (5).

3. The measuring apparatus (1) according to claim 1, wherein the first tube (5) is made of a plastic material and the second tube (6) is made of a metal material.

4. The measuring apparatus (1) according to claim 3, wherein the first tube (5) is made of PEEK or PTFE.

5. The measuring apparatus (1) according to claim 3 or 4, wherein the second tube (6) is made of stainless steel.

## Patentansprüche

1. Messgerät (1) zur Inline-Messung des Homogenisierungsgrades eines flüssigen Produkts in einer Dispergiereinheit (100), wobei das Messgerät Folgendes umfasst:
- eine rohrförmige Vorrichtung (2), die an einem Ausgang (100b) der Dispergiereinheit (100) angeordnet werden kann, wobei die rohrförmige Vorrichtung (2) einen internen Hohlraum (3) für den Durchlass des von der Dispergiereinheit (100) aufgenommenen flüssigen Produkts aufweist, wobei die rohrförmige Vorrichtung (2) ein erstes Rohr (5) und ein zweites Rohr (6) umfasst, das erste Rohr (5) koaxial zu dem zweiten Rohr (6) und innenseitig in diesem angeordnet ist, der interne Hohlraum (3) durch eine interne Oberfläche (5a) des ersten Rohrs (5) begrenzt ist, die erste rohrförmige Vorrichtung (2) ferner einen Wärmetauscher umfasst, bei dem es sich um eine Leitung (8) für den Durchlass einer Kühlflüssigkeit handelt, und die Leitung (8) durch die rohrförmige Vorrichtung (2) über deren gesamte Länge führt und einen Hauptteilbereich aufweist, der parallel zu einer Längsachse (A-A) der rohrförmigen Vorrichtung (2) verläuft;
eine Vielzahl von Ultraschallsensoren (4), die entlang der Länge der rohrförmigen Vorrichtung (2) verteilt sind und zwischen dem ersten Rohr (5) und dem zweiten Rohr (6) angeordnet sind, wobei das erste Rohr (5) eine Vielzahl von Aufnahmen (7) zum Aufnehmen der Ultraschallsensoren (4) aufweist, die Aufnahmen (7) als nach innen gerichtete Ausnehmungen einer externen Oberfläche (5b) des ersten Rohrs (5) ausgebildet sind und die Vielzahl von Ultraschallsensoren durch den Wärmetauscher abgekühlt werden,
wobei der interne Hohlraum (3) einen variablen Durchmesser über die Länge der rohrförmigen Vorrichtung (2) hinweg aufweist, der interne Hohlraum (3) mindestens einen ersten Teilbereich (31), aufweisend eine zusammenlaufende Form von einem Einlass (2a) der rohrförmigen Vorrichtung (2) in Richtung eines Auslasses (2b) der rohrförmigen Vorrichtung (2), aufweist, der interne Hohlraum (3) auch einen zweiten Teilbereich (32) aufweist, der von dem ersten Teilbereich (31) ausgeht und an dem Auslass (2b) der rohrförmigen Vorrichtung (2) endet, der zweite Abschnitt (32) eine sich verjüngende Entwicklung von dem Auslass (2b) der rohrförmigen Vorrichtung (2) in Richtung des ersten Teilbereichs (31) aufweist und der zweite Teilbereich (32) aus einem stumpfkegelförmigen Abschnitt (320), der den ersten Teilbereich (31) mit dem Auslass (2b) der rohrförmigen Vorrichtung (2) verbindet, besteht,
wobei der erste Teilbereich (31) des internen Hohlraums (3) durch eine Vielzahl von im Wesentlichen zylindrischen Abschnitten (310) gebildet ist, die durch stumpfkegelförmige Abschnitte (311) zusammengefügt sind, und die zylindrischen Abschnitte (310) entsprechende Durchmesser aufweisen, die von dem Einlass (2a) in Richtung des Auslasses (2b) der rohrförmigen Vorrichtung (2) abnehmen, wobei jeder zylindrische Abschnitt (310) entweder
- mit einem der Ultraschallsensoren (4) gekuppelt ist, der sowohl als Sender als auch als Empfänger von Ultraschallwellen wirkt und innerhalb einer der Aufnahmen (7) angeordnet ist, die in einem Teilabschnitt des ersten Rohrs (5) ausgebildet ist, der den entsprechenden zylindrischen Abschnitt (310) umgibt, oder
- mit einem der Ultraschallsensoren (4), der als Empfänger von Ultraschallwellen wirkt, und einem anderen der Ultraschallsensoren (14), der als Sender von Ultraschallwellen wirkt, gekuppelt ist, wobei der Sender und der Empfänger jeweils in zwei Aufnahmen (7) der Aufnahmen (7) angeordnet sind, die in Bezug auf den internen Hohlraum (3) entgegengesetzt platziert sind und in einem Teilabschnitt des ersten Rohrs (5) ausgebildet sind, der den entsprechenden zylindrischen Abschnitt (310) umgibt, aber auf anderen Seiten in Bezug auf die Strömung des flüssigen Produkts.

2. Messgerät (1) nach Anspruch 1, wobei die Ultraschallsensoren (4) an dem ersten Rohr (5) angeklebt sind.

3. Messgerät (1) nach Anspruch 1, wobei das erste Rohr (5) aus Kunststoffmaterial besteht und das zweite Rohr (6) aus Metallmaterial besteht.

4. Messgerät (1) nach Anspruch 3, wobei das erste Rohr (5) aus PEEK oder PTFE besteht.

5. Messgerät (1) nach Anspruch 3 oder 4, wobei das zweite Rohr (6) aus rostfreiem Stahl besteht.

## Revendications

1. Appareil de mesure (1) pour mesure en ligne du degré d'homogénéisation d'un produit fluide dans une unité de dispersion (100), ledit appareil de mesure comprenant :
- un dispositif tubulaire (2) pouvant être agencé à une sortie (100b) de l'unité de dispersion (100), ledit dispositif tubulaire (2) ayant une cavité interne (3) pour le passage du produit fluide reçu de l'unité de dispersion (100), ledit dispositif tubulaire (2) comprenant un premier tube (5) et un second tube (6), le premier tube (5) étant coaxial et agencé intérieurement à l'intérieur du second tube (6), ladite cavité interne (3) étant délimitée par une surface interne (5a) du premier tube (5), ledit dispositif tubulaire (2) comprenant en outre un échangeur de chaleur qui est un conduit (8) pour le passage d'un fluide de refroidissement, ledit conduit (8) passant à travers le dispositif tubulaire (2) sur toute sa longueur et ayant une voie principale qui est parallèle à un axe longitudinal (A-A) du dispositif tubulaire (2) ;
une pluralité de capteurs à ultrasons (4) distribués le long de la longueur du dispositif tubulaire (2) et étant agencés entre le premier tube (5) et le second tube (6), ledit premier tube (5) ayant une pluralité de logements (7) pour recevoir lesdits capteurs à ultrasons (4), lesdits logements (7) étant obtenus comme des évidements vers l'intérieur d'une surface externe (5b) du premier tube (5), ladite pluralité de capteurs à ultrasons étant refroidie par l'échangeur de chaleur,
dans lequel ladite cavité interne (3) a un diamètre variable sur la longueur du dispositif tubulaire (2), la cavité interne (3) comportant au moins une première voie (31) ayant une forme convergente depuis une entrée (2a) du dispositif tubulaire (2) vers une sortie (2b) du dispositif tubulaire (2), ladite cavité interne (3) comportant également une seconde voie (32) provenant de la première voie (31) et se terminant à la sortie (2b) du dispositif tubulaire (2), ladite seconde voie (32) ayant un développement effilé depuis la sortie (2b) du dispositif tubulaire (2) vers ladite première voie (31), ladite seconde voie (32) consistant en une portion frusto-conique (320) reliant la première voie (31) à la sortie (2b) du dispositif tubulaire (2),
dans lequel la première voie (31) de la cavité interne (3) est formée par une pluralité de portions substantiellement cylindriques (310) qui sont jointes par des portions frusto-coniques (311), lesdites portions cylindriques (310) ayant des diamètres correspondants qui diminuent de l'entrée (2a) vers la sortie (2b) du dispositif tubulaire (2), chaque portion cylindrique (310) étant soit :
- couplée à l'un desdits capteurs à ultrasons (4) agissant à la fois comme émetteur et récepteur d'ondes ultrasonores et agencé à l'intérieur de l'un desdits logements (7) obtenus dans une section du premier tube (5) entourant ladite portion cylindrique correspondante (310), soit
- couplé à l'un desdits capteurs à ultrasons (4) agissant comme récepteur d'ondes ultrasonores et à un autre desdits capteurs à ultrasons (14) agissant comme émetteur d'ondes ultrasonores, ledit émetteur et ledit récepteur étant respectivement agencés dans deux logements (7) desdits logements (7) situés à l'opposé par rapport à la cavité interne (3) et étant obtenus dans une section du premier tube (5) entourant la portion cylindrique correspondante (310), mais sur des côtés différents par rapport à l'écoulement du produit fluide.

2. Appareil de mesure (1) selon la revendication 1, dans lequel les capteurs à ultrasons (4) sont collés au premier tube (5).

3. Appareil de mesure (1) selon la revendication 1, dans lequel le premier tube (5) est réalisé en un matériau plastique et le second tube (6) est réalisé en un matériau métallique.

4. Appareil de mesure (1) selon la revendication 3, dans lequel le premier tube (5) est réalisé en PEEK ou en PTFE.

5. Appareil de mesure (1) selon la revendication 3 ou 4, dans lequel le second tube (6) est réalisé en acier inoxydable.
